# EUROPEAN PATENT SPECIFICATION

(11) **EP 1 980 219 B1**
(45) Date of publication and mention of the grant of the patent: **02.09.2009**
(21) Application number: 08102172.7
(22) Date of filing: 29.02.2008
(51) Int. Cl.: A61F 2/16

(54) **Lens delivery system cartridge and method of manufacture**
Linseneinführungskarktuschensystem und Verfahren zur Herstellung
Cartouche de système de mise en place de lentille et procédé de fabrication

(30) Priority: 11.04.2007 US 734084
(43) Date of publication of application: 15.10.2008
(73) Proprietor: Alcon, Inc., 6331 Hünenberg (CH)
(72) Inventor: Downer, David A., Fort Worth, TX 76137 (US); Brown, Kyle, Fort Worth, TX 76132 (US); Muchala, Sushant, Kennedale, TX 76060 (US); Cam Tran, Tu, Grapevine, TX 76051 (US); Yan, Dengzhu, Arlington, TX 76017 (US)
(74) Representative: Hanna, Peter William Derek

(56) References cited:
- WO-A-96/20662
- JP-A- 2000 025 073
- US-A- 5 444 183
- US-A- 6 083 231

## Description

This invention relates to intraocular lenses (IOLs) and more particularly to cartridges for use with devices use to inject IOLs into an eye.

### Background of the Invention

The human eye in its simplest terms functions to provide vision by transmitting and refracting light through a clear outer portion called the cornea, and further focusing the image by way of the lens onto the retina at the back of the eye. The quality of the focused image depends on many factors including the size, shape and length of the eye, and the shape and transparency of the cornea and lens.

When trauma, age or disease cause the lens to become less transparent, vision deteriorates because of the diminished light which can be transmitted to the retina. This deficiency in the lens of the eye is medically known as a cataract. The treatment for this condition is surgical removal of the lens and implantation of an artificial lens or IOL.
While early IOLs were made from hard plastic, such as polymethylmethacrylate (PMMA), soft, foldable IOLs made from silicone, soft acrylics and hydrogels have become increasingly popular because of the ability to fold or roll these soft lenses and insert them through a smaller incision. Several methods of rolling or folding the lenses are used. One popular method is an injector cartridge that folds the lenses and provides a relatively small diameter lumen through which the lens may be pushed into the eye, usually by a soft tip plunger. The most commonly used injector cartridge design is illustrated in US. Patent No. 4,681,102 (Bartell), and includes a split, longitudinally hinged cartridge. Similar designs are illustrated in US. Patent Nos. 5,494,484 and 5,499,987 (Feingold) and 5,616,148 and 5,620,450 (Eagles, et al.). In an attempt to avoid the claims of US. Patent No. 4,681,102, several solid cartridges have been investigated, see for example US. Patent No. 5,275,604 (Rheinish, et al.) and 5,653,715 (Reich, et al.).

These prior art devices were intended to inject an IOL into the posterior chamber of an aphakic eye through a relatively large (approximately 3.0 mm or larger) incision. Surgical techniques and IOLs have been developed that allow the entire surgical procedure to be performed through much smaller incisions, 2.4 mm and smaller. Such small incisions require that the IOL be compressed very tightly, and that the nozzle used on the injection cartridge have very thin walls. The combination of a tightly compressed lens traveling through a very thin walled nozzle often results in the nozzle splitting during use.

Documents US-A-6 083 231 and WO-A-96 20662 disclose the preamble of claim 1.

Accordingly, a need continues to exist for an intraocular lens injection cartridge capable of injection an IOL through a relatively small incision.

### Brief Summary of the Invention

The present invention improves upon prior art by providing a cartridge for an IOL delivery system that has a straight, thinned walled distal nozzle, in accordance with claims which follow. The transition region between the tapered folding portion of the cartridge and the nozzle contains reinforcing gussets to help prevent splitting of the cartridge. Flow leaders in the nozzle direct the flow of material during molding at the 12:00 o'clock position, positioning the weld line of the flow front at the 6:00 o'clock position.

It is accordingly an objective of the present invention to provide a cartridge for a lens delivery system that has a straight, thinned walled distal nozzle.

It is a further objective of the present invention to provide a cartridge for a lens delivery system that contains reinforcing gussets to help prevent splitting of the cartridge.

Other objectives, features and advantages of the present invention will become apparent with reference to the drawings, and the following description of the drawings and claims.

### Brief Description of the Drawings

FIG. 1 is an enlarged top perspective view of the lens delivery system cartridge of the present invention.
FIG. 2 is an enlarged top perspective view of the distal nozzle of the lens delivery system cartridge of the present invention.
FIG. 3 is an enlarged side elevational view of the distal nozzle of the lens delivery system cartridge of the present invention.

### Detailed Description of the Preferred Embodiments

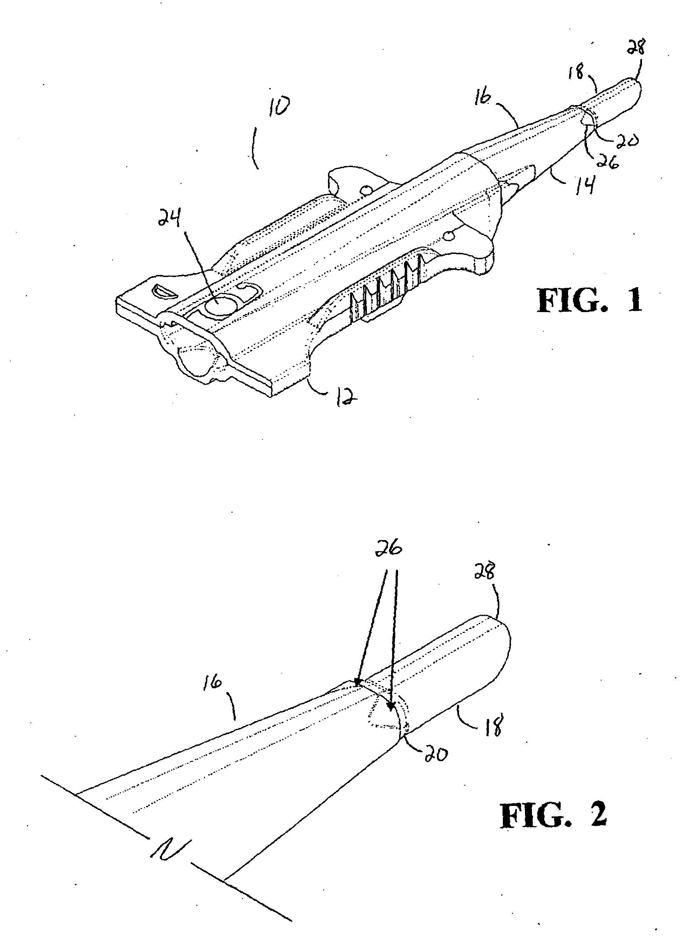

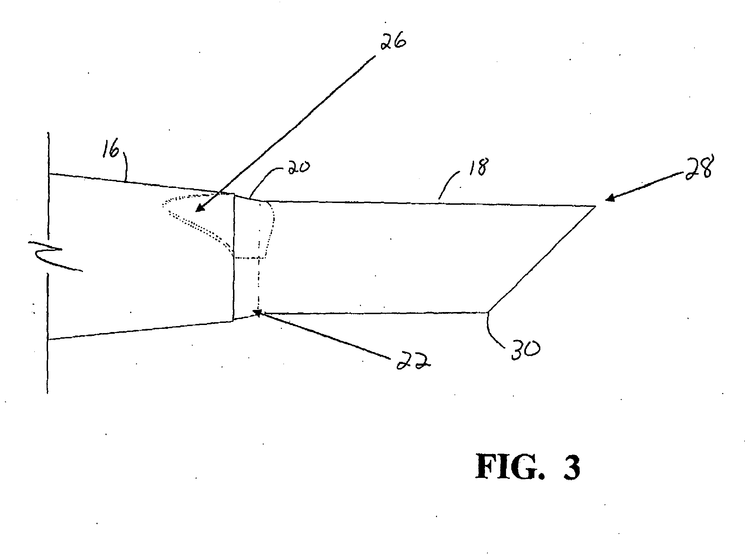

As best seen in FIGS. 1-3, lens cartridge 10 of the present invention generally includes body 12 and nozzle 14. Cartridge 10 can be molded from any suitable thermoplastic, such as polypropylene, and the thermoplastic may contain a lubricity enhancing agent such as those disclosed in U.S. Pat. No. 5,716,364. Nozzle 14 may be integrally formed with body 12. Nozzle 14 has a tapered portion 16 and a straight portion 18. Straight portion 18 has very thin walls, on the order and between 0.07 mm and 0.17 mm thick and preferably is tubular and of substantially constant round, oval or elliptical cross-section, with a cross-sectional area of between around 1.0 mm² to around 2.6 mm². Straight portion 18 is preferably on the order of 3 mm to 5 mm long so as to allow cartridge 10 to deliver IOL 24 inside the capsular bag without tapered portion 16 entering the incision. Such a construction does not enlarge the incision during IOL insertion. Transition area 20 between tapered portion 16 and straight portion 18 contain reinforcing gusset 22. Reinforcing gusset 22 helps prevent splitting of nozzle 14 at transition area 20.

Located on the proximal side of transition area 20, at the distal end of tapered portion 16 are raised flow leaders 26. Flow leaders 26 help direct the flow of material during injection molding of cartridge 10 to 12:00 o'clock position 28, then downwardly around straight portion 18, causing the weld line to form at 6:00 o'clock position 30. The inventors have surprisingly discovered that maximum stress in straight portion 18 occurs around 12:00 o'clock position 28, while minimum stress in straight portion 18 occurs around 6:00 o'clock position 30. Directing the weld line of the material flow to 6:00 o'clock position 30 places the weakest position of straight portion 18 at the location of minimal stress, thereby helping to reduce splitting of straight portion 18 during IOL injection.

While certain embodiments of the present invention have been described above, these descriptions are given for purposes of illustration and explanation. Variations, changes, modifications and departures from the systems and methods disclosed above may be adopted without departure from the scope of the present invention.

## Claims

1. An intraocular lens delivery system cartridge (10), comprising:
a) a body (12),
b) a tubular nozzle (14) connected to the body and projecting distally from the body, the nozzle having a proximal tapered portion (16) and a distal straight portion (18); and
c) a transition area (20) between the tapered portion (16) and the straight portion (18) of the nozzle, the transition area defining a gusset (22), **characterized by** the tapered portion (16) further comprises a plurality of flow leaders (26).

2. A method of manufacturing an intraocular lens delivery system cartridge (10), of claim 1, by injection moulding comprising the steps of:
a) forming a body (12);
b) forming a tubular nozzle (14) having a proximal tapered portion (16) and a distal straight portion (18), the nozzle being connected to the body and projecting distally from the body; and
c) forming a transition area (20) between the tapered portion (16) and the straight portion (18) of the nozzle, and forming a reinforcing gusset (22) at the transition area (20), and wherein raised flow leaders (26) are provided in the mould to help direct the flow of material during injection moulding of the cartridge (10) to a 12:00 o'clock position (28), then downwardly around straight portion (18), so as to cause the weld line of the flow front to form at a 6:00 o'clock position (30).

## Patentansprüche

1. Kartusche (10) für ein Intraokularlinsen-Einführsystem, mit:
a) einem Körper (12),
b) einer mit dem Körper verbundenen rohrförmigen Düse (14), die vom Körper aus in distaler Richtung herausragt, wobei die Düse einen proximalen konischen Abschnitt (16) und einen distalen geraden Abschnitt (18) hat; und
c) einem Übergangsbereich (20) zwischen dem konischen Abschnitt (16) und dem geraden Abschnitt (18) der Düse, wobei der Übergangsbereich ein Einsatzstück (22) definiert,
**dadurch gekennzeichnet, dass**
der konische Abschnitt (16) ferner eine Mehrzahl Strömungsführungen (26) aufweist.

2. Verfahren zum Herstellen einer Kartusche (10) für ein Intraokularlinsen-Einführsystem gemäß Anspruch 1 durch Spritzgießen, mit den Schritten:
a) Formen eines Körpers (12);
b) Formen einer rohrförmigen Düse (14) mit einem proximalen konischen Abschnitt (16) und einem distalen geraden Abschnitt (18), wobei die Düse mit dem Körper verbunden ist und vom Körper in distaler Richtung herausragt; und
c) Formen eines Übergangsbereichs (20) zwischen dem konischen Abschnitt (16) und dem geraden Abschnitt (18) der Düse und Ausbilden eines Verstärkungs-Einsatzstücks (22) am Übergangsbereich (20), wobei erhabene Strömungsführungen (26) im Formteil vorgesehen sind, um dazu beizutragen, den Materialfluss während des Spritzgießens der Kartusche (10) zur 12:00-Uhr-Position (28) und dann nach unten um den geraden Abschnitt (18) zu richten, damit bewirkt wird, dass sich die Schweißlinie der Strömungsfront in 6:00-Uhr-Position (30) bildet.

## Revendications

1. Cartouche de système de mise en place de lentille intraoculaire (10), comprenant :
a) un corps (12),
b) une buse tubulaire (14) reliée au corps et faisant saillie de manière distale à partir du corps, la buse ayant une partie conique proximale (16) et une partie droite distale (18) ; et
c) une zone de transition (20) entre la partie conique (16) et la partie droite (18) de la buse, la zone de transition définissant un gousset (22), **caractérisée en ce que** la partie conique (16) comporte en outre une pluralité de guides d'écoulement (26).

2. Procédé de fabrication d'une cartouche de système de mise en place de lentille intraoculaire (10) selon la revendication 1, par moulage par injection, comportant les étapes consistant à :
a) former un corps (12) ;
b) former une buse tubulaire (14) ayant une partie conique proximale (16) et une partie droite distale (18), la buse étant reliée au corps et faisant saillie de manière distale à partir du corps ; et
c) former une zone de transition (20) entre la partie conique (16) et la partie droite (18) de la buse, et former un gousset de renforcement (22) au niveau de la zone de transition (20), des guides d'écoulement surélevés (26) étant fournis dans le moule afin d'aider à diriger l'écoulement de matériau pendant le moulage par injection de la cartouche (10) vers une position située à 12 heures (28), puis vers le bas autour de la partie droite (18), afin de provoquer la formation de ligne de soudure du front d'écoulement au niveau d'une position située à 6 heures (30).
